# EUROPEAN PATENT APPLICATION

(11) **EP 3 130 659 A1**
(43) Date of publication of application: **15.02.2017**
(21) Application number: 15776072.9
(22) Date of filing: 09.04.2015
(51) Int. Cl.: C12M 1/00

(54) **OCTAGONAL PILLAR-SHAPED CELL CULTURE CONTAINER**

(30) Priority: 10.04.2014 JP 2014081429
(71) Applicant: Animal Stem Cell, Koganei-shi, Tokyo 184-0012 (JP)
(72) Inventor: NAGAIKE Kazuhiro, Koganei-shi Tokyo 184-0012 (JP); SUZUKI Toshinori, Koganei-shi Tokyo 184-0012 (JP)
(74) Representative: V.O.
(86) International application number: PCT/JP2015/061152
(87) International publication number: WO 2015/156367

(57) **Abstract**

The present invention addresses the problem of providing a means, whereby mesenchymal stem cells or adherent cells can be mass-cultured, while monitoring the cell state and strictly maintaining and controlling the same during the course of culture, to obtain cells in an amount as required for treatment of a disease, and can be homogeneously, economically, and efficiently harvested. The present invention also addresses the problem of providing a mass culture container capable of complying with a variety of demands for setting culture conditions; for example, coating of culture surface(s), combination(s) of various types of cells to be adhered to the culture surface(s), and so forth. To resolve these problems, provided is an octagonal-prism-shaped cell culture container having a closed bottom end portion and having an opening for liquid at a top end portion which is mutually opposed thereto, and in which the respective surfaces that form said octagonal prism thereof are planar, being a cell culture container in which mutually opposed pairs of surfaces among the respective surfaces that form said octagonal prism are parallel, and having a structure such that the closed bottom end portion thereof is conical in shape or is in the shape of an octagonal pyramid. The octagonal-prism-shaped cell culture container is usable as a means for homogeneous, economic and efficient mass culture.

## Description

### TECHNICAL FIELD

The present invention relates to a container for culture of cells; more specifically, the present invention relates to an octagonal-prism-shaped cell culture container, having a closed bottom end portion and having an opening for liquid at a top end portion which is mutually opposed thereto, such as may be used for homogeneous and affordable culture and growth of any of various types of cells including mesenchymal cells, mesenchymal stem cell, and other such adherent cells.

### BACKGROUND ART

Cells of many varieties that have been isolated from living organisms are cultured under artificial conditions intended to replicate the environment of the living organisms, transfection of genes is carried out where necessary, and large quantities of these cells, as well as of the various factors produced by these cells, are obtained and used to treat various diseases. Included among such cells are adherent cells such as mesenchymal stem cells, mesenchymal cells, and fibroblasts which have properties permitting growth following adhesion to a location constituting a scaffold as a result of interaction with various molecules between the cells and the scaffold. Such adherent cells may be grown through use of a container for culture of adherent cells that might take the form of a cell culture dish or cell culture flask having a culture surface that has been imparted with adherent characteristics.

To be permit use for medical treatment as mentioned above, large quantities of cells must be cultured, and the cells, as well as factors and other such components produced by the cells, must be recovered. However, because the cell culture container ordinarily comprises a single planar culture surface, there is a limit to the area of the culture surface that is available for growth of cells.

To make mass culture of cells possible, strategies may therefore be adopted in which the size of the cell culture container that is used is increased, or the number of containers used for culture is increased. However, because there is typically a limit to the amount of space that can be allotted for culture, there has been the problem that it has not been possible to adequately increase the number containers that are used to carry out culture.

And even where space capable of being allotted for culture is available, the size of the cell culture container can be increased, or the number of cell culture containers can be increased, the number of operations required for culture increases dramatically in direct proportion to the increase in the size of the container and the increase in the number of containers. For this reason, preparation of large quantities of homogeneous cells has entailed much inconvenience, there has been increased risk of contamination, and cleverness and expertise have proven necessary for carrying out work operations.

Moreover, there has also been the problem that increase in the scale with which culture is carried out necessitates employment of large amounts of culture medium and of the blood serum and various factors that are added thereto, as well as of various types of culture equipment including pipettes and centrifuge tubes, causing dramatic increase in cost.

Thus, there has been a need to solve the various aforementioned problems that arise in connection with mass culture in the context of medical treatment and in the context of manufacturing.

While various stratagems have been devised as stratagems to solve the foregoing problems, as a means suited to mass production and employing equipment and so forth, a roller bottle has been proposed which is an object having roughly cylindrical shape, the portion having such roughly cylindrical shape being made to rotate continuously about the axis thereof during use, and which is such that the entire inside surface of said roughly cylindrical shape is made to serve as culture surface. Moreover, to increase the surface area of the culture surface to which adherent cells can adhere, a roller bottle has also been proposed that is manufactured so as to have corrugations in the axial direction at the portion of said roller bottle which has roughly cylindrical shape (Patent Reference No. 1).

However, in a container having the foregoing structure, problems arise in that, because the culture medium is present at the below of the inside surface of the cylindrical portion, the surface area of the liquid surface of the culture medium is less than the surface area of the culture surface, and there is insufficient exchange of air with the layer of air above the culture medium.

As a container that would simultaneously allow culture area to be maximized and the difference between the surface area at the liquid surface of the culture medium and the culture surface to be minimized, a cell culture bottle has therefore also been proposed, an essential constitutional element of which is that the cylindrical portion of the bottle is molded so that the cross-sectional shape of the roller bottle that had been roughly cylindrical is made instead to be in the shape of a Reuleaux polygon, i.e., so as to produce a structure having curved sides and an odd number of rounded corners (Patent Reference No. 2).

However, with each of the foregoing respective roller bottles, because the culture surface must be curved so as to achieve a structure permitting mass culture of cells to be carried out, it is impossible to acquire information about the number of cells, cell shape, and/or other such detailed information while culture is in progress by means employing microscopic examination, and so it is impossible to control the state of cells while culture is in progress. This being the case, before optimal culture conditions could be established, it has been necessary any number of times during the course of operations to stop operations in mid-culture, to recover the adhered cells that have grown and evaluate cell number and cell quality, with the result that a great many cells have gone to waste so that conditions for carrying out mass culture could be established. And this has been a particularly large problem when the initial number of cells is extremely small or when cells that cannot easily be obtained are employed, a situation being created in which it has been difficult enough just to achieve the conditions that will permit mass culture, much less reach the point where this can be applied to medical treatment, which was the reason for carrying out such culture in the first place, and so there has been a need for improvement in technique.

In the case of mesenchymal stem cells, which are adherent cells that may be employed for treatment of disease, the undifferentiated state that they possess by virtue of their being stem cells must be strictly maintained. For this reason, to obtain the number of cells required for application to medical treatment, it is necessary when performing culture for growth of mesenchymal stem cells that this be carried out in conjunction with monitoring for strict control of the state of progress of culture so that cell number, cell density, the state of the culture medium, and so forth are maintained in constant fashion. In particular, to strictly maintain the undifferentiated state possessed by stem cells, it is important that strict control be carried out so that the number of cells and the density of cells adhering to the culture surface are constant at the start of culture.

However, where a conventional culture dish or culture bottle is employed to carry out culture and growth while strictly maintaining the undifferentiated state possessed by stem cells, because this creates difficulties when the scale with which culture is carried out is increased as described in the foregoing paragraph, and because there have been increases in cost, there has been a need for stratagems that might be employed with respect to the culture means.

There have therefore been problems when the roller bottles for mass culture of cells of the conventional type which are described at the foregoing respective patent references have been employed. To achieve mass culture, such mass culture roller bottles have been designed so as to have as an essential structural element thereof the fact that they are cylindrical, are cylindrical but with corrugations in the axial direction, or are in the shape of a Reuleaux polygon. For this reason, because each of these bottles has a structure such that the culture surface thereof is curved, it has been the case with these that there have been differences in the depth of the culture medium from the culture medium surface to the culture surface at the interior of the bottle. This being the case, at the start of culture, concentration of cells tends to be high at the central portion of the bottle where the depth of the culture medium from the culture medium surface to the culture surface at the interior of the bottle is deep, and there is a tendency for cells that have not yet adhered to accumulate and for clusters of cells to form at the lower portion of the curved culture surface. This being the case, because strict control cannot be carried out so that the number of cells and the density of cells adhering to the culture surface at the start of culture is constant, these are not suitable for culture of mesenchymal stem cells. To obtain homogeneous cells, formation of clusters of cells is not preferred not only for mesenchymal stem cells but also for other adherent cells. Moreover, because the culture surface is curved, microscopic examination cannot be employed to monitor cell state while culture is in progress, and it is impossible to ascertain cell density during culture or the degree of uniformity thereof, or whether clusters of cells have formed, until cells are dislodged from the bottle and are recovered.

It has therefore conventionally been difficult to cause growth of homogeneous mesenchymal stem cells in which the undifferentiated state possessed by stem cells has been strictly maintained to proceed to a point where these are present in a quantity as required for use in medical treatment and are available for clinical use.

And to prevent this, if one were to attempt to cause cells to adhere uniformly, so that cells do not settle in nonuniform fashion and form clusters of cells, it will be necessary to cause the start of culture to take place under rotating conditions. But if this is done, because a force will be exerted on the cells due to movement of the culture surface and the culture medium at the time of rotation, causing reduction in the force of adhesion possessed by the cells with respect to the culture surface, this will make it impossible to employ any cells except those which possess a comparatively large force of adhesion, reducing degrees of freedom available when selecting cells for use in medical treatment.

Furthermore, to obtain cells that possess specific properties, there are situations in which it is necessary to carry out coculture together with other cells, in which case, to permit use for medical treatment, stratagems might be need to be devised to remove the cocultured cells or to reduce as much as possible the extent of their admixture therewith. However, where the foregoing conventional culture dish, culture bottle, roller bottle is employed, it has been difficult to cause only those cells that were cocultured therewith to be removed from the cells for medical treatment that were grown in large quantities or to cause reduction in admixture thereof by the cells that were cocultured therewith. Furthermore, with conventional coculture containers, the cost of the container is very expensive, such that difficulty would be encountered if one were to attempt to use a large enough number of conventional coculture containers to permit cells to be cultured in quantities large enough to permit them to be employed for medical treatment.

### PRIOR ART REFERENCES

### PATENT REFERENCES

Patent Reference No. 1: Japanese Patent Application Publication Kokoku No. H06[1994]-61256
Patent Reference No. 2: Specification of U.S. Patent No. 5866419

### SUMMARY OF INVENTION

### PROBLEM TO BE SOLVED BY INVENTION

A problem to be solved by the present invention is to carry out mass culture of mesenchymal stem cells used in treatment of disease with strict maintenance and control while monitoring the undifferentiated state possessed by the stem cells during the course of culture to such an extent as will make it possible to obtain cells in quantities as required for treatment of disease, and to homogeneously, affordably, and efficiently recover said cells.

Furthermore, a problem to be solved by the present invention, to obtain adherent cells used in treatment of disease and/or factors produced by adherent cells, is to carry out mass culture while monitoring the state of the cells during the course of culture to such an extent as will make it possible to obtain cells in quantities as required for treatment of disease, and to homogeneously, affordably, and efficiently recover said cells and/or said factors.

Another problem to be solved by the present invention is to eliminate the dramatic increase in cost due to increase in the quantity of pipettes and centrifuge tubes used for recovery of cultured cells, the required quantity of which increases in accompaniment to increase in the scale with which culture is carried out, and to provide an affordable means for carrying out mass culture.

Furthermore, another problem to be solved by the present invention is to provide a container for mass culture permitting accommodation of the desire to satisfy a wide range of culture conditions, such as any of various combinations with respect to coating treatment(s) employed at culture surface(s), type(s) of cells made to adhere to culture surface(s), and so forth during mass culture.

### MEANS FOR SOLVING PROBLEM

A first means in accordance with the present invention for solving the foregoing problems is a cell culture container comprising an octagonal-prism-shaped cell culture container having a closed bottom end portion and having an opening for liquid at a top end portion mutually opposed thereto, wherein respective surfaces forming said octagonal prism are planar, and wherein two mutually opposed surfaces among the respective surfaces forming said octagonal prism are parallel.

A second means in accordance with the present invention for solving the foregoing problems is a cell culture container according to the foregoing first means wherein a neck portion further extends from the opening for liquid which is open at the top end portion, said neck portion having external threads for receiving a lid having internal threads.

A third means in accordance with the present invention for solving the foregoing problems is a cell culture container according to any one of the foregoing first and second means wherein at an outside circumferential region of a base portion toward the opening for liquid at the neck portion there is a protruding portion for securing a cavity-possessing member or a gripping member for holding the cell culture container.

A fourth means in accordance with the present invention for solving the foregoing problems is a cell culture container according to any one of the foregoing first through third means wherein the closed bottom end portion is conical in shape or is in a shape of an octagonal pyramid.

A fifth means in accordance with the present invention for solving the foregoing problems is a cell culture container according to any one of the foregoing first through fourth means wherein gradations are present on an outside surface of the cell culture container.

A sixth means in accordance with the present invention for solving the foregoing problems is a cell culture container according to any one of the foregoing first through fifth means wherein a marking is present on an outside of any desired surface among the respective surfaces that form the octagonal prism of the cell culture container.

A seventh means in accordance with the present invention for solving the foregoing problems is a cell culture container according to any one of the foregoing first through sixth means wherein gelatin, extracellular matrix, and/or a polycationic substance is used to modify culture surfaces, increasing adhesion of cells thereto, at interiors of respective surfaces forming said octagonal prism of the cell culture container.

### BENEFIT OF THE INVENTION

Employment of a cell culture container which is a means in accordance with the present invention that is an octagonal-prism-shaped cell culture container having a closed bottom end portion and having an opening for liquid at a top end portion which is mutually opposed thereto, and in which the respective surfaces that form said octagonal prism thereof are planar, being a cell culture container in which mutually opposed pairs of surfaces among the respective surfaces that form said octagonal prism are parallel, makes it possible to ascertain the state of cells as they are being cultured, makes it possible to achieve conditions suitable for mass culture even where the initial number of cells is extremely small or even where cells that cannot easily be obtained are employed, and, because the culture surfaces are planar, makes it possible to preserve cell density uniformity, to maintain an undifferentiated state such as exists with cells such as mesenchymal stem cells that may be employed for treatment of disease, and/or to otherwise homogeneously and affordably carry out mass culture and growth of adherent cells for which particular cell properties must be maintained. Moreover, causing the cell culture container to have a structure such that the closed bottom end portion thereof is conical in shape or is in the shape of an octagonal pyramid makes it possible for it to also possess the ability to be used as a centrifugal separation tube, permitting achievement of even further reductions in cost.

### BRIEF DESCRIPTION OF THE DRAWINGS

[FIG. 1] Drawing showing an example of an octagonal-prism-shaped cell culture container in accordance with the present invention.
[FIG. 2] Sectional drawing of an example of an octagonal-prism-shaped cell culture container in accordance with the present invention.
[FIG. 3] Drawing showing an example in which a lid is used to seal an octagonal-prism-shaped cell culture container in accordance with the present invention.
[FIG. 4] Drawing illustrating how an octagonal-prism-shaped cell culture container in accordance with the present invention might be used to carry out culture.
[FIG. 5] Photograph of a container showing how gelatin coating treatment had been carried out at every other culture surface in an octagonal-prism-shaped cell culture container in accordance with the present invention.

### EMBODIMENTS FOR CARRYING OUT THE INVENTION

Below, embodiments for carrying out the present invention are described as appropriate with reference to the drawings.

### (Materials)

There is no particular limitation with regard to the material(s) that may be employed for formation of the member(s) that make up the lid and cell culture container of the present invention, it being possible to use materials such as are ordinarily employed in the context of cell culture.

While resin material(s), and inorganic material(s) such as glass or quartz or the like, or metal, may for example be employed as the foregoing material(s), it is preferred based on considerations which include ease of manufacturing, cost reduction, and ease of ascertaining the state of cell culture that resin material(s) be employed.

In addition, as the foregoing resin material(s), polyethylene terephthalate resin, polystyrene resin, polyester resin, polyethylene resin, polypropylene resin, ABS resin, Nylon, acrylic resin, fluorocarbon resin, polycarbonate resin, polyurethane resin, methylpentene resin, phenolic resin, melamine resin, epoxy resin, polyvinyl chloride resin, and/or or other such resin material(s), and/or resin material(s) containing at least one of the foregoing that has been subjected to surface hydrophilicity treatment may be used. In addition, as said resin material(s), it is preferred based on considerations which include ease of manufacturing, cost reduction, and ease of ascertaining the state of cell culture that polyethylene terephthalate resin and/or polystyrene resin be employed.

(Shape of Octagonal-Prism-Shaped Cell Culture Container) Octagonal-prism-shaped cell culture container 1 of the present invention has an octagonal-prism-shaped cell culture container structure having a closed bottom end portion 2 and an opening 3 for liquid at a top end portion that is mutually opposed thereto. In addition, the respective surfaces that form the foregoing octagonal prism thereof are planar, the structure being such that mutually opposed pairs of surfaces among the respective surfaces that form the octagonal prism are more or less parallel.

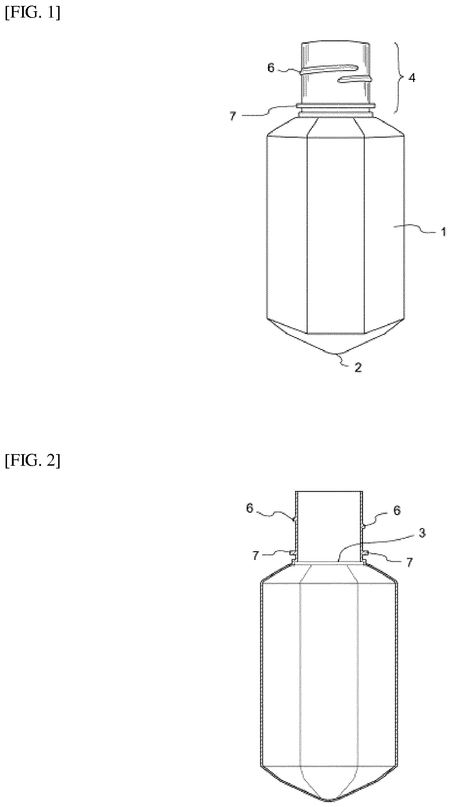

Furthermore, to permit use as a culture centrifuge container, it may also be made to have a structure such as will allow it to be used not only for cell culture but also as a centrifugal separation tube in which the foregoing closed bottom end portion 2 is conical in shape or is in the shape of an octagonal pyramid. (FIG. 1 and FIG. 2.)

In addition, neck portion 4 further extends from opening 2 for liquid which is open at the top end portion, said neck portion having external threads 6 for receiving lid 5 which has internal threads.

Moreover, in a structure for facilitating mechanical control of roll-tube culture, at the outside circumferential region of the base portion toward the opening for liquid at the foregoing neck portion 4 there is a protruding portion 7 for securing gripping member(s) or cavity-possessing member(s) for holding said octagonal-prism-shaped cell culture container upright, holding said octagonal-prism-shaped cell culture container horizontal, and for holding said octagonal-prism-shaped cell culture container so as to cause opening 2 for liquid to be inclined in downward fashion so as to permit discharge by decantation of the culture medium with which the interior of the container is filled.

Furthermore, the structure may be made such that gradations are applied as desired to the outside surface of the foregoing cell culture container 1, said gradations permitting the amount of culture medium and the amounts of cells within the culture container to be estimated by visual inspection. In addition, the structure may be made such that marking(s) are moreover applied to the outside(s) of any desired surface(s) among the respective surfaces that form the octagonal prism of the foregoing cell culture container so as to permit the respective culture surfaces to be respectively distinguished.

Note that it is necessary that the foregoing cell culture container be 1., of thickness such as will deliver a wall pressure on the order of that such as will allow sufficient strength to be possessed thereby when filled with culture medium and culture is carried out. Moreover, where the structure is such that the bottom end portion is conical in shape or is in the shape of an octagonal pyramid so as to permit use not only as a culture container but also for cell recovery as a centrifugal separation tube, the cell culture container/centrifugation tube should be made to have thickness such as will provide strength permitting it to sufficiently withstand centrifugal separation as necessary for recovery of cells.

### (Manufacturing Operation)

The cell culture container of the present invention may be manufactured using a mold prepared so as to permit molding in the shape of the foregoing octagonal-prism-shaped cell culture container 1; and where the foregoing resin material(s) is/are employed, it may be formed in integral fashion by blow molding.

Depending on the type of cells being cultured, there may be situations in which it is necessary while culture is in progress to employ a phase contrast microscope or the like to examine and monitor in detailed fashion the state of the cells being cultured. So as to permit microscopic examination to be carried out satisfactorily and the state of the cells to be monitored in detailed fashion while culture is in progress, prior to manufacture of the container, treatment for obtaining a specular surface at portions of the mold that will form the culture surfaces might therefore be carried out or any of various other such techniques such as will cause the culture surfaces of the octagonal-prism-shaped cell culture container of the present invention to be in a low-distortion state suitable for examination by means of an optical microscope, fluorescence microscope, phase contrast microscope, or any of various other such microscopes might be carried out.

### (Modification of Culture Surfaces)

Moreover, as component(s) for carrying out modification of culture surface(s) for increasing adherence of cells to culture surface(s) of octagonal-prism-shaped cell culture container 1 of the present invention, gelatin, extracellular matrix, and/or polycationic substance(s) may be employed as substance(s) applied to surface(s) constituting culture surface(s). So long as they are such as to permit employment for cell culture, there is no particular limitation with regard to the type of gelatin, extracellular matrix, and/or polycationic substance(s). Collagen, laminin, fibronectin, and/or the like may be employed as extracellular matrix; polylysine, polyethylene imine, polyornithine, and/or the like may be employed as polycationic substance(s).

Furthermore, the foregoing respective components may be applied to location(s) such as might be expected to provide increased adherence to culture surface(s), it being possible to apply these to all eight culture surfaces of the octagonal-prism-shaped cell culture container 1 of the present invention, it being possible to apply these to every other surface for a total of four surfaces, it being possible to apply these to only desired surface(s), and it moreover being possible to apply different component(s) to different surface(s).

### (Operation for Applying Marking(s) to Outer Surface(s) of Cell Culture Container)

Because the octagonal-prism-shaped cell culture container 1 of the present invention has eight culture surfaces, marking(s) for distinguishing the respective surfaces may be applied to the outside of the container so as to make it possible to distinguish the respective culture surfaces through employment of equipment for recognition thereof or through visual identification thereof from the exterior. The marking(s) which are employed may be selected from among characters, symbols, barcodes, patterns, and/or any other desired item(s), it being possible to print these thereon using ink or to scribe these thereon by means of a laser or etching. Furthermore, the desired marking(s) may be applied in advance to said container mold so that the item which is manufactured will have marking(s) on the surface(s) of the cell culture container.

By causing marking(s) to be applied at necessary location(s) on the exterior portion of the octagonal-prism-shaped cell culture container 1 of the present invention as described above, and by using this in combination with device(s) that recognize said marking(s), it will be possible to cause the position(s) of the culture container and of culture surface(s) at said container to be accurately recognized by processing device(s) that carry out rotational control of the octagonal-prism-shaped cell culture container of the present invention, permitting increase in the degrees of freedom with which rotational control may be carried out. Furthermore, even where rotational control of the octagonal-prism-shaped cell culture container of the present invention is carried out by the operator himself or herself, it will be possible to recognize the respective positions of the culture surfaces at said container, permitting increase in the accuracy of operations.

Furthermore, by causing octagonal-prism-shaped cell culture container 1 of the present invention to have a structure such that the container bottom portion 2 is conical in shape or is in the shape of an octagonal pyramid, it will be possible to achieve a structure that can also be used as a centrifugal separation tube that permits cells which have been dislodged from the culture surfaces of the container to be subjected to centrifugal separation in a centrifugal separator. This may therefore be made such that gradations are applied to the outside thereof at the location of the conical shape or octagonal pyramid so as to make it possible to visually determine the amount of culture medium and/or cells that have collected in the form of a pellet at the location of the conical shape or octagonal pyramid at the bottom portion of the container following centrifugal separation. The gradations may be applied thereto by printing these thereon using ink or by scribing these thereon by means of a laser or etching. Furthermore, the gradations may be applied in advance to the container mold so that the item which is manufactured will have gradations on the surface of the container.

By employing transparent resin material(s), glass, quartz, and/or other such inorganic material(s) and/or the like in the cell culture container, because it will be possible before carrying out centrifugation to measure from the exterior of the cell culture container the turbidity of the culture medium in which the cells have been suspended, it will be possible through extrapolation based on the value of turbidity that was measured to quantify the number of cells that have been suspended within the culture medium, and it will be possible following centrifugation to measure the size of the pellet by visual inspection or through employment of camera(s) or sensor device(s) and to roughly quantify the number of cells within the pellet that has been recovered.

### (Sterilization Operation)

The octagonal-prism-shaped cell culture container 1 of the present invention may be provided in the form of a sterilized item. There is no particular limitation with regard to the method(s) for sterilization of the cell culture container, it being possible to use methods such as are ordinarily employed as methods for sterilization of cell culture containers. As said sterilization method, methods which include gamma irradiation sterilization, e-beam sterilization, radiation sterilization, ethylene oxide gas sterilization, ultraviolet irradiation sterilization, hydrogen peroxide sterilization, and ethanol sterilization, may, for example, be employed. In addition, as said sterilization method, it is preferred based on considerations which include ease of manufacturing and cost reduction that, to facilitate mass production of the cell culture container, e-beam sterilization or gamma irradiation sterilization be employed. While it is preferred that e-beam sterilization be carried out to such a degree as will not cause degradation of the cell culture container, and it is preferred that the irradiative energy during gamma irradiation sterilization be within a range that is up to on the order of 5 kGy to 30 kGy so as to cause sterilization to be carried out to such a degree as will not cause degradation of the cell culture container, suitable sterilization method(s) may be selected and employed in accordance with the cells used.

### WORKING EXAMPLES

Indicated below is a working example in which an octagonal-prism-shaped cell culture container 1 in accordance with the present invention is manufactured and used. The present invention is not to be limited in any way by these descriptions.

### (Working Example 1)

An octagonal-prism-shaped cell culture container 1 in accordance with the present invention was manufactured. A mold for molding the octagonal-prism-shaped cell culture container 1 and the lid 5 used therewith was fabricated. Said mold was subjected to treatment for obtaining a specular surface so that culture surfaces would be smooth, and provisions were made to cause gradations to be applied to the outside of the bottom portion of the octagonal-prism-shaped cell culture container 1 at the location of the octagonal pyramid following molding.

Polyethylene terephthalate resin was employed to carry out blow molding using the foregoing mold to mold an octagonal-prism-shaped cell culture container 1 in integral fashion. Ink was used to apply markings to the round culture exterior of the container so as to make it possible for the respective culture surfaces to be recognized. Lid 5 was molded in similar fashion. The manufactured octagonal-prism-shaped cell culture container 1 and a portion of the lid 5 were sterilized by e-beam sterilization.

### (Working Example 2)

A 2% pigment-containing gelatin solution was prepared.

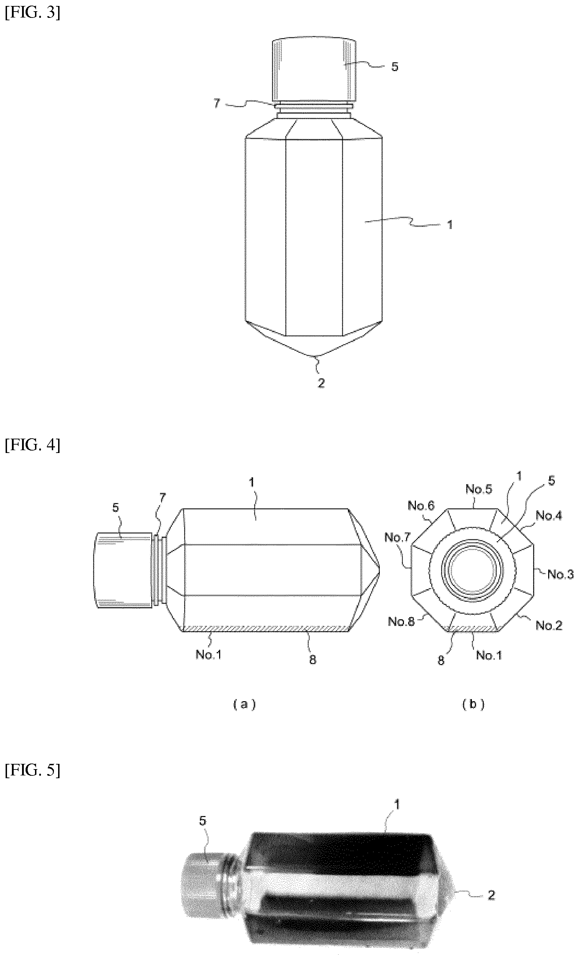

The octagonal-prism-shaped cell culture container 1 was made to lie on its side such that one of the surfaces (the surface marked as No. 1 in FIG. 4) among the culture surfaces forming the octagonal prism thereof was made to serve as bottom surface, and an amount of the 2% pigment-containing gelatin solution sufficient to cover said bottom surface was poured thereinto by way of opening 3 for liquid disposed at the top end portion of the container, and this was thereafter left undisturbed for a prescribed time so as to cause the gelatin solution to adhere to Culture Surface No. 1. Note that the pigment was added so as to facilitate easy determination from the exterior of whether coating treatment by gelatin had occurred uniformly.

With the octagonal-prism-shaped cell culture container 1 still lying on its side, a procedure was carried out in which said container was rotated in clockwise fashion about the axis of the octagonal prism thereof so as to cause the culture surface (the surface marked as No. 3 in FIG. 4) two surfaces away in the clockwise direction from the culture surface that had been made to serve as the bottom surface to become the new bottom surface. In addition, an amount of the 2% pigment-containing gelatin solution sufficient to cover said bottom surface was poured thereinto by way of opening 3 for liquid disposed at the top end portion of the container, and this was left undisturbed for a prescribed time so as to cause the gelatin solution to adhere to Culture Surface No. 1. The foregoing procedure was repeated, and the gelatin solution was made to adhere to the surfaces marked as No. 5 and No. 7 in FIG. 4.

Using the foregoing procedure, it was possible to cause every other surface for a total of four of the culture surfaces among the culture surfaces forming the octagonal prism to be subjected to coating treatment (FIG. 5).

It was clear that the octagonal-prism-shaped cell culture container 1 of the present invention, in which the respective culture surfaces that formed the octagonal prism thereof were planar and in which mutually opposed pairs of surfaces among the respective surfaces that formed the octagonal prism were more or less parallel, made it possible to carry out coating treatment of respective culture surfaces in a manner that was convenient and that permitted treatment of any desired surface(s) thereamong.

Moreover, based on the fact that it was possible to easily carry out treatment of every other surface as described above, it was clear that the octagonal-prism-shaped cell culture container 1 of the present invention also made it possible to easily perform a procedure in which cells desired to be cultured are made to adhere only to the desired surface(s) thereamong.

### (Working Example 3)

Adipose-derived mesenchymal stem cells from canine subcutaneous adipose tissue were separated and cultured. All experiments and procedures were carried out in accordance with guidelines formulated by the Institutional Care Use Committee.

Dog(s) were anesthetized and procedures carried out to obtain subcutaneous adipose tissue. Subcutaneous adipose tissue was subjected to enzymatic treatment, this was washed with 1 x PBS buffer solution (dilution ratio 1:1; v/v) and was thereafter redispersed in 1 x PBS buffer solution, following which an operation was carried out in which this was made to pass multiple times through a syringe. The buffer solution was discarded, following which adipose-derived mesenchymal stem cells that had adhered to the interior wall of the syringe and within the hollow of the needle were recovered in the presence of 1 x PBS buffer solution to which EDTA had freshly been added. The adipose-derived mesenchymal stem cells that were recovered were washed with 1 x PBS buffer solution, following which a prescribed number of cells were resuspended in culture medium to which FBS had been added for carrying out culture.

Culture medium 8 in which the foregoing adipose-derived mesenchymal stem cells were suspended was gently poured into the interior of octagonal-prism-shaped cell culture container 1 of the present invention by way of opening 3 for liquid disposed at the top end portion of the container, and lid 5 was placed on neck portion 4 of the container. Care was taken not to overtighten lid 5, and care was taken to maintain sufficient ventilation when octagonal-prism-shaped cell culture container 1 was left undisturbed within a cell culture incubator.

The octagonal-prism-shaped cell culture container 1 was made to lie on its side such that one of the surfaces (the surface marked as No. 1 in FIG. 4) among the culture surfaces forming the octagonal prism thereof was made to serve as bottom surface, and this was left undisturbed within a cell culture incubator that had been set to temperature, humidity, and to carbon dioxide and oxygen concentrations as appropriate for adipose-derived mesenchymal stem cells.

At regular time intervals, a procedure was carried out in which, with the octagonal-prism-shaped cell culture container 1 still lying on its side, said container was rotated in clockwise fashion about the axis of the octagonal prism thereof so as to cause the culture surface (the surface marked as No. 2 in FIG. 4) adjacent direction to the culture surface that had been made to serve as the bottom surface to become the new bottom surface. This was repeated at regular time intervals so as to bring the surfaces through one full rotation from No. 3 through No. 8.

After the octagonal-prism-shaped cell culture container had been made to undergo one full rotation in clockwise fashion about the axis of the octagonal prism, a phase contrast microscope was used to examine all eight culture surfaces, and observations were made with respect to the amounts of cells adhering thereto as well as the manner in which they were distributed thereacross.

As a result, it was found that at each of the eight culture surfaces of the octagonal-prism-shaped cell culture container 1 in accordance with the present invention, the adipose-derived mesenchymal stem cells had uniformly adhered to that culture surface. In addition, in accompaniment to rotation in clockwise fashion about the axis of the octagonal prism, a tendency toward reduced cell density with increased time left undisturbed was observed from Culture Surface No. 1 to No. 8. However, for shorter times left undisturbed, while there were fewer cells adhering to respective culture surfaces, no difference in cell density was observed.

It was thus learned that the octagonal-prism-shaped cell culture container of the present invention was such that the density with which adipose-derived mesenchymal stem cells were initially made to adhere could be made more or less constant by optimizing the time that culture surface(s) were left undisturbed in accompaniment to rotation in clockwise fashion about the axis of the octagonal prism.

The foregoing octagonal-prism-shaped cell culture container 1 on which adipose-derived mesenchymal stem cells had been made to adhere on the respective surfaces thereof was left undisturbed within a cell culture incubator, and microscopic examination was carried out after culture was continued as said container was further rotated in clockwise fashion about the axis of the octagonal prism thereof at regular time intervals. In addition, lid 5 was opened, treatment was carried out to dislodge the adipose-derived mesenchymal stem cells that had adhered to the culture surfaces, following which the volume of liquid suspension and the number of cells within the liquid suspension were measured.

In addition, centrifugal separation processing was furthermore carried out, and said container was inclined in diagonal fashion, causing a pellet of the adipose-derived mesenchymal stem cells to be left behind, and the unwanted culture supernatant was discarded by decantation by way of opening 3 for liquid disposed at the top end portion of said container. The pellet of adipose-derived mesenchymal stem cells was thereafter recovered

As a result, it was found that there was satisfactory increase in the number of adipose-derived mesenchymal stem cells.

### (Comparative Example)

As a comparative example, culture medium in which the foregoing adipose-derived mesenchymal stem cells had been suspended was added to a conventional roller bottle, and roll-tube culture was carried out. Because the culture surfaces of the roller bottle were of curved shape, microscopic observation of the entire culture surface could not easily be accomplished. After the cells had then been dislodged therefrom, the number of cells was measured, the liquid suspension of cells was transferred to centrifuge tubes and centrifugal separation processing was carried out, and the adipose-derived mesenchymal stem cells were recovered therefrom in the form of a pellet. As a result, the relative increase in the number of cells was less than was the case with the octagonal-prism-shaped cell culture container of the present invention.

### INDUSTRIAL UTILITY

Employment, as a container for mass culture of adherent cells, of the foregoing cell culture container which is a means in accordance with the present invention that is an octagonal-prism-shaped cell culture container having a closed bottom end portion and having an opening for liquid at a top end portion which is mutually opposed thereto, and in which the respective surfaces that form said octagonal prism thereof are planar, being a cell culture container in which mutually opposed pairs of surfaces among the respective surfaces that form said octagonal prism are parallel, and that moreover has a structure such that the closed bottom end portion thereof is conical in shape or is in the shape of an octagonal pyramid makes it possible to ascertain the state of the cells as they are being cultured, and makes it possible to achieve conditions suitable for mass culture even where the initial number of cells is extremely small or even where cells that cannot easily be obtained are employed.

Furthermore, because the culture surfaces of the culture container are planar, this makes it possible to preserve cell density uniformity, to maintain an undifferentiated state such as exists with cells such as mesenchymal stem cells that may be employed for treatment of disease, and/or to otherwise homogeneously and affordably carry out mass culture and growth of adherent cells for which particular cell properties must be maintained.

Moreover, it makes it possible to provide a container for mass culture of cells in which it is possible to vary the coating treatment or the cells adhering thereto such that there is a different coating treatment at and/or different cells adhering to different respective culture surfaces thereof, permitting accommodation of the desire to satisfy a wide range of culture conditions.

### EXPLANATION OF REFERENCE NUMERALS

- 1: Octagonal-prism-shaped cell culture container
- 2: Bottom end portion
- 3: Opening for liquid
- 4: Neck portion
- 5: Lid having internal threads
- 6: External threads
- 7: Protruding portion
- 8: Culture medium in which cells have been suspended

## Claims

1. A cell culture container comprising an octagonal-prism-shaped cell culture container having a closed bottom end portion and having an opening for liquid at a top end portion mutually opposed thereto, wherein respective surfaces forming said octagonal prism are planar, and wherein two mutually opposed surfaces among the respective surfaces forming said octagonal prism are parallel.

2. A cell culture container according to claim 1 wherein a neck portion further extends from the opening for liquid which is open at the top end portion, said neck portion having external threads for receiving a lid having internal threads.

3. A cell culture container according to any one of claim 1 and claim 2 wherein at an outside circumferential region of a base portion toward the opening for liquid at the neck portion there is a protruding portion for securing a cavity-possessing member or a gripping member for holding the cell culture container.

4. A cell culture container according to any one of claim 1 through claim 3 wherein the closed bottom end portion is conical in shape or is in a shape of an octagonal pyramid.

5. A cell culture container according to any one of claim 1 through claim 4 wherein gradations are present on an outside surface of the cell culture container.

6. A cell culture container according to any one of claim 1 through claim 5 wherein a marking is present on an outside of any desired surface among the respective surfaces that form the octagonal prism of the cell culture container.

7. A cell culture container according to any one of claim 1 through claim 6 wherein the respective surfaces that form the octagonal prism of the cell culture container comprise quartz, glass, and/or a resin material having transparency.

8. A cell culture container according to any one of claim 1 through claim 7 wherein gelatin, extracellular matrix, and/or a polycationic substance is used to modify culture surfaces, increasing adhesion of cells thereto, at interiors of respective surfaces forming said octagonal prism of the cell culture container.
